(19) Europäisches Patentamt
European Patent Office
Office européen des brevets

(11) **EP 4 169 506 B1**

(12) **EUROPEAN PATENT SPECIFICATION**

(45) Date of publication and mention
of the grant of the patent:
**04.03.2026 Bulletin 2026/10**

(21) Application number: **22202451.5**

(22) Date of filing: **19.10.2022**

(51) International Patent Classification (IPC):
*A61K 9/107* (2006.01)    *A61K 9/14* (2006.01)
*A61K 9/16* (2006.01)    *A61K 31/164* (2006.01)
*A23L 33/12* (2016.01)    *A61P 1/00* (2006.01)
*A61P 15/00* (2006.01)    *A61P 15/02* (2006.01)
*A61P 19/00* (2006.01)    *A61P 19/02* (2006.01)
*A61P 21/00* (2006.01)    *A61P 25/02* (2006.01)
*A61P 29/00* (2006.01)    *A61K 9/00* (2006.01)
*A23L 33/00* (2016.01)    *A61K 31/00* (2006.01)

(52) Cooperative Patent Classification (CPC):
(C-Sets available)
A61K 9/107; A23L 33/12; A61K 9/0095;
A61K 9/143; A61K 9/145; A61K 9/146;
A61K 9/1611; A61K 9/1617; A61K 9/1623;
A61K 9/1652; A61K 31/047; A61K 31/164;
A61K 31/202; A61P 1/00; A61P 15/00;    (Cont.)

(54) **PALMITOYLETHANOLAMIDE-BASED COMPOSITION IN THE FORM OF POWDERS WITH HIGH ENTERIC BIOACCESSIBILITY**

PALMITOYLETHANOLAMID BASIERTE PUDER-ZUBEREITUNG MIT HOHER ENTERISCHER BIOVERFÜGBARKEIT

COMPOSITION A BASE DE PALMITOYLETHANOLAMIDE SOUS FORME DE POUDRE A HAUTE BIOACCESSIBILITE ENTERIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **20.10.2021 IT 202100026924**

(43) Date of publication of application:
**26.04.2023 Bulletin 2023/17**

(73) Proprietor: **Geofarma S.r.l.**
**70042 Mola di Bari (BA) (IT)**

(72) Inventors:
• **Fratter, Andrea**
**30030 Olmo di Martellago VE (IT)**
• **Pellizzato, Marzia**
**31050 Vedelago TV (IT)**
• **Campanile, Maria Grazia**
**70042 Mola di Bari BA (IT)**
• **Lattaruli, Leonardo**
**70042 Mola di Bari BA (IT)**

(74) Representative: **Perani & Partners S.p.A.**
**Corso Europa, 15**
**20122 Milano (IT)**

(56) References cited:
EP-A1- 3 785 699       WO-A1-2020/024009
WO-A2-2011/161501      WO-A2-2017/212447
US-A1- 2020 330 423

• B.V. RAJESH ET AL: "LIPID BASED SELF-EMULSIFYING DRUG DELIVERY SYSTEM (SEDDS) FOR POORLY WATER-SOLUBLE DRUGS: A REVIEW", JOURNAL OF GLOBAL PHARMA TECHNOLOGY, vol. 2, 1 March 2010 (2010-03-01), pages 47 - 55, XP055025732
• SINGH BHUPINDER ET AL: "Self-Emulsifying Drug Delivery Systems (SEDDS): Formulation Development, Characterization, and Applications", CRITICAL REVIEWS IN THERAPEUTIC DRUG CARRIER SYSTEMS, vol. 26, no. 5, 1 January 2009 (2009-01-01), US, pages 427 - 521, XP055925582, ISSN: 0743-4863, DOI: 10.1615/CritRevTherDrugCarrierSyst.v26.i5.10

- **SAXENA SHIVANGI ET AL: "Lipid Excipients in Self Emulsifying Drug Delivery Systems", 10 August 2013 (2013-08-10), XP055925583, Retrieved from the Internet <URL:https://www. alliedacademies.org/articles/ lipid-excipients-in-self-emulsifying-drug-deliver y-systems.pdf>**

(52) Cooperative Patent Classification (CPC): (Cont.)
    **A61P 15/02; A61P 19/00; A61P 19/02; A61P 21/00; A61P 25/02; A61P 29/00**

C-Sets
**A61K 31/047, A61K 2300/00;
A61K 31/164, A61K 2300/00;
A61K 31/202, A61K 2300/00**

## Description

### FIELD OF THE INVENTION

The present invention is placed in the technical field of formulations for conveying palmitoylethanolamide, intended for oral intake, as defined by the claims.

### BACKGROUND ART

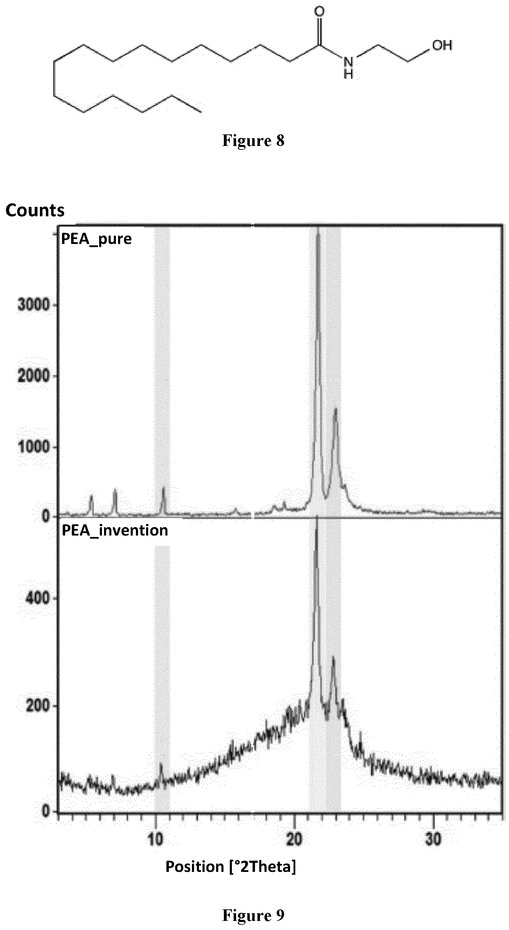

Palmitoylethanolamide (PEA) is, chemically, the ethanolamide of palmitic acid (Figure 8), an endogenous factor belonging to the class of nuclear factor agonists. From a pharmacodynamic point of view, PEA cannot be considered a true endocannabinoid, lacking specificity for the CB1 and CB2 receptors even though its interaction with the cannabinoid-like G-coupled receptors GPR55 and GPR119 has been shown (Godlewski G. Prostaglandins & Other Lipid Mediators. 89 (3-4): 105-11). Its pharmacological action is extrinsic through binding to specific nuclear receptors, which results in a wide variety of biological functions related to chronic pain and inflammation. The main pharmacodynamic target appears to be the PPAR-$\alpha$ receptors.

PEA is a biosimilar molecule of the endocannabinoid anandamide (Devane et al 1992) and this analogy justifies the pharmacological and clinical evidence on interactions between PEA and the endogenous endocannabinoid system (Jonsson et al 2001; Petrosino et al 2010; Skaper and Di Marzo 2012). Such evidence has favoured subsequent pharmacological and clinical investigations which, overall, have confirmed the efficacy of PEA in the modulation of inflammatory phenomena.

In particular, the strongest clinical evidence indicates a significant efficacy of PEA in the resolution of inflammation, when associated with some specific conditions such as neuronal inflammation (peripheral neuritis and under arthropathy) and pelvic inflammation with consequent pain reduction (Jaggar et al 1998; Farquhar-Smith et al 2001; Capasso et al 2001; Farquhar-Smith et al 2002; Haller et al 2006; Pessina et al 2014; Borrelli et al 2015). It is important to note that, unlike anti-inflammatory drugs, PEA preserves the analgesic and anti-inflammatory effect even in the event of chronic and/or neuropathic pain (Jaggar et al 1998; Capasso et al 2001; Costa et al 2008; De Filippis et al 2010; De Filippis et al 2011; Luongo et al 2013; Bettoni et al 2013; Di Cesare Mannelli et al 2013).

Given the well-known pharmacological activities of PEA and the results on preclinical models, a series of clinical studies have followed over the years which have employed PEA in micronized and ultra-micronized forms and have investigated its effectiveness on pathological conditions associated with neuro-inflammation and chronic and/or neuropathic pain (Skaper et al 2014; Freitag and Miller 2014). In particular, a double-blind, randomized, 2-dose controlled study of PEA versus placebo showed the dose-dependent analgesic effect of micronized PEA (m-PEA) in patients with compressive lumbosciatalgia (Guida et al 2010).

Under the same conditions, micronized PEA therapy has been shown to favour a reduction in the use of NSAIDs, realizing the strategy of a combined use, also in order to reduce the important adverse effects of the latter in chronic use (Canteri et al 2010). The results obtained in patients with lumbosciatalgia have been confirmed in a large number of patients with chronic pain associated with different pathological conditions, such as radiculopathy, osteoarthritis, spinal surgical failure syndrome, post herpetic neuropathy, diabetic neuropathy and cancer pain (Gatti et al 2012; Marini et al 2012).

WO2017/212447A2 discloses oral powder formulations, e.g. for filling capsules, wherein the powder comprises a self-emulsifying and gastroresistant fat matrix adsorbed onto a solid phase comprising a polyol (such as mannitol, xylitol, sorbitol or isomalt).

*Problem of the prior art*

The use of PEA in medical therapy, net of its pharmacological properties and the clinical evidence mentioned above, is still limited, mainly due to the low bioavailability of the molecule, essentially attributable to its very poor enteric bioaccessibility. This phenomenon, well documented and in turn linked to the poor water solubility of the molecule, strongly limits the bioaccessible enteric fraction which is the indispensable condition for the entry into circulation of the substance.

Other absorption pathways, such as through the oral mucosa, have not produced particularly positive results: due to the high lipophilicity of the molecule, the elective pathway of the substance for physiological absorption remains the gastrointestinal one.

The technologies and the main technological efforts aimed at increasing the bioavailability of PEA have been dedicated, in recent years, to increasing the contact surface between the PEA and the absorbent enteric epithelium, through micronization and ultra-micronization processes in order to reduce the particle size of the substance so as to increase, precisely, the total contact surface between it and the epithelium.

Despite scientific works having been published which indicate some improvement in human pharmacokinetic parameters of PEA in micronized and ultra-micronized form, following oral intake (Linda Gabrielsson et al Br J Clin Pharmacol (2016) 82 932-942, Impellizzeri et al. J Neuroinflammation. 2014 Aug 28; 11:136, Emanuela Stochino Loi et al. Int J Womens Health. 2019 Aug 12;11:443-449), an effective technological system capable of increasing the bioaccessibility of PEA is not yet available.

## SUMMARY OF THE INVENTION

The Applicant has identified a self-emulsifying lipid matrix, as defined by the claims, designed to divert PEA into the digestive tract in a form such as to give rise to a liquid emulsion in contact with gastro-enteric fluids, starting from a suitably processed powder.

Therefore, a first object of the present invention is a solid composition, in powder form, comprising palmitoylethanolamide (PEA) as an active ingredient, the solid composition being characterized in that it comprises

a) a lipid-surfactant phase including the PEA and containing

-    at least one medium chain triglyceride (MTC)
-    at least one mono and diglyceride of fatty acids.
-    at least one mono- or poly-unsaturated fatty acid, or precursors thereof
-    at least one hydrophilic surfactant,

b) a hydrophilic phase in the form of granules containing glucose polymers and/or polyols, on which the lipid-surfactant phase is absorbed, as defined by the claims.

A further object of the present invention is a process for the preparation of the aforesaid solid composition, comprising the steps of:

i) preparing a lipid-surfactant mixture comprising at least one medium-chain triglyceride (MTC); at least one mono- and diglyceride of fatty acids; at least one mono- or poly-unsaturated fatty acid or a precursor thereof; at least one hydrophilic surfactant;
ii) dissolving the lipid-surfactant mixture until a hot, liquid and transparent lipid-surfactant mixture is obtained;
iii) adding the PEA to the hot lipid-surfactant mixture, under stirring, until completely dissolved, to obtain the hot lipid-surfactant phase (a);
iv) preparing the hydrophilic phase in the form of granules (b) and adding the hot lipid-surfactant phase (a), under stirring, maintaining the temperature, to obtain a hot precursor of the solid composition, in powder form;
v) maintaining the hot precursor of the solid composition, in powder form, under stirring and cooling to room temperature, to obtain the solid composition, in powder form;
vi) sieving the solid composition, in powder form, to a particle size between 565 nm and 1,233 nm.

*Advantages of the invention*

The self-emulsifying lipid matrix allows to convey PEA in solid compositions, improving the bioaccessibility thereof through an emulsification phenomenon thereof in gastro-enteric liquids.

It should be noted that mono and diglycerides of fatty acids play a key role in the composition, since they represent the fraction of the self-emulsifying lipid matrix which, solidifying at room temperature together with the triglyceride, mono- or poly-unsaturated fatty acid and the surfactant, "harnesses" the active ingredient (in this case the PEA), creating the conditions for the formation of a solid. Such a solid, in contact with gastro-enteric secretions, generates mixed micelles together with bile salts, simulating the action of pancreatic lipases: *in vivo*, in fact, it occurs that the products of hydrolysis of food triglycerides, generated by the action of pancreatic lipases, form mixed micelles with bile salts, which are then absorbed by the enterocytes. The Applicant therefore considers that the improved bioaccessibility of PEA is bound, at least in part, to the in vivo formation of the aforementioned mixed micelles.

Furthermore, such a self-emulsifying lipid matrix is able to change the state of aggregation of the active ingredient from crystalline to amorphous, generating an even more effective dispersion of PEA (in terms of enteric absorption).

It should be noted that prior to being dispersed in the hot lipid phase, the PEA is characterized by a solid form and crystalline state of aggregation. Analysing the PEA formulated in accordance with the invention by means of differential scanning calorimetry techniques, coupled to an X-ray diffractometry, it is detected that the active substance dispersed in the self-emulsifying lipid matrix, despite being in predominantly amorphous form, maintains an identifiable crystalline structure detectable by characteristic diffraction peaks (Figure 9).

Since the solid composition of the invention is prepared hot, it is believed that the crystallinity of the PEA favours the recrystallization of the powder at room temperature. In other words, the production process of the composition does not require a cooling step at low temperatures (below room temperature) in order to achieve the recrystallization of the powder. This is a considerable advantage in industrial terms, not only from an operational point of view, but also from that of energy consumption.

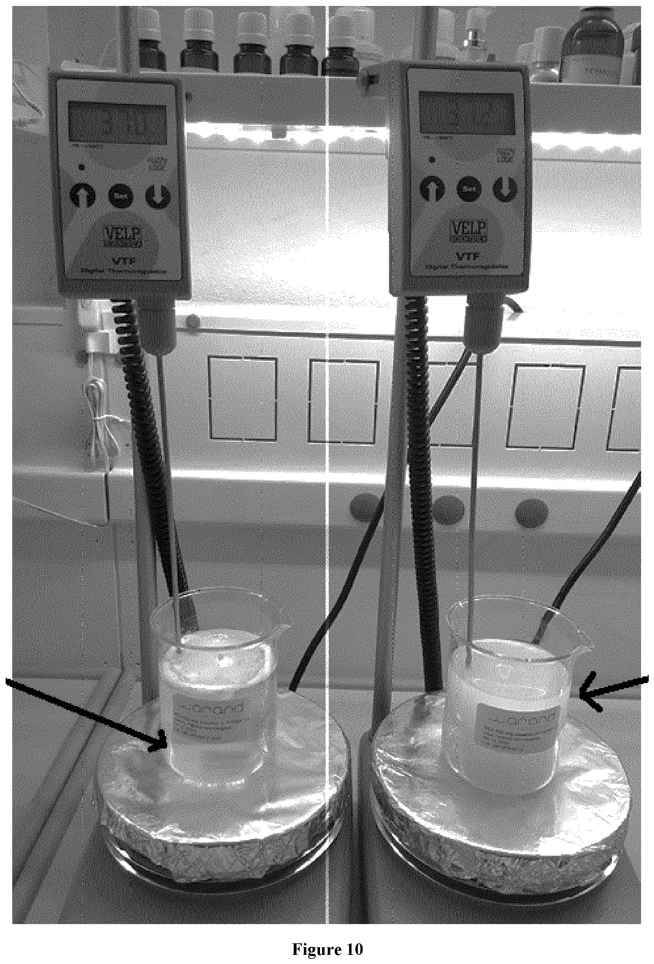

Of particular importance is the phenomenon whereby the PEA formulated according to the invention gives rise, already at the gastric level, to an emulsion in the absence of solid particulate matter (suspension), uniformly dispersed, despite the crystallinity mentioned above (Figure 10).

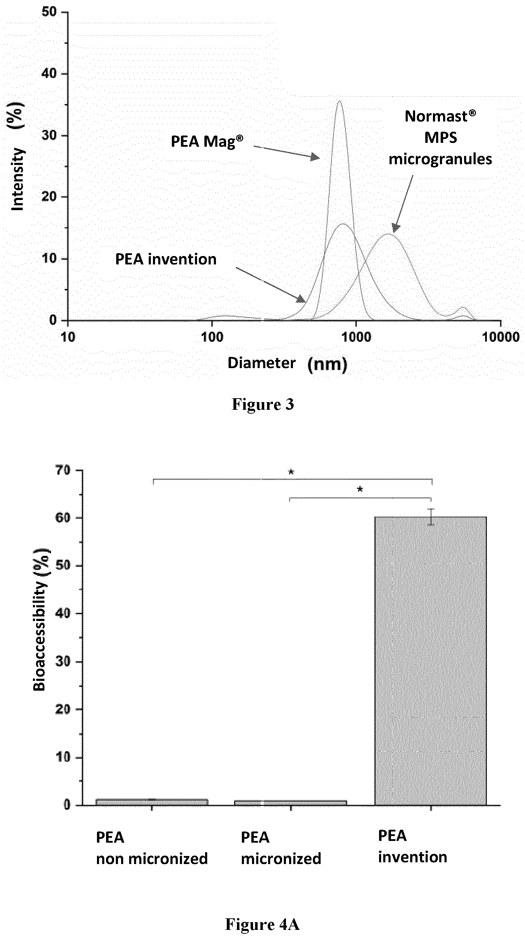

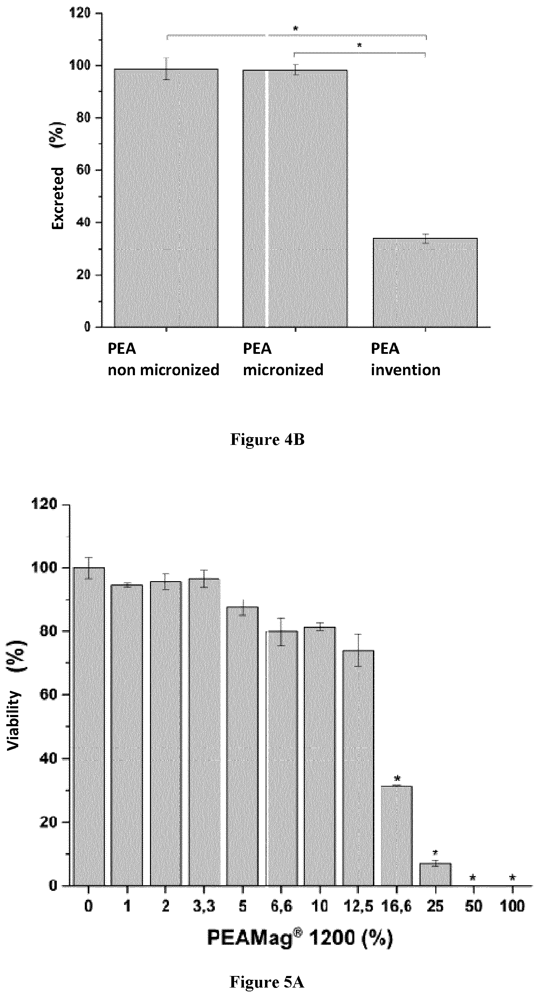

Furthermore, from what has emerged from the bioaccessibility analyses performed (Examples - section IV.3.3), it is clear that the PEA formulated in accordance with the invention is much more bioaccessible than the PEA conveyed at the same dosage in micronized or non-micronized form. The bioaccessible fractions of PEA in the micronized and non-micronized form are substantially equivalent, not exceeding 1% of the tested dose. In contrast, the PEA formulated in accordance with the present invention demonstrates a bioaccessibility between 50 and 70%, preferably equal to about 60% of the tested dose (Figure 4).

## DESCRIPTION OF THE FIGURES

*Figure 1*. Representative image of the in vitro model of intestinal epithelium, with the Caco-2 cell monolayer grown on a Transwell® insert.

*Figure 2*. (A) Electron micrograph of enterocytes forming the Caco-2 monolayer with the microvilli and occluding junctions highlighted; (B) Confocal microscopy images highlighting the occluding junctions (red) and cell nuclei (blue).

*Figure 3*. Dimensional distribution of PEAMag®, normast® MPS microgranules and PEA of the invention measured by DLS at the end of the digestive process in vitro.

*Figure 4*. Bioaccessibility (A) and excreted fraction (B) of the PEA present in the three formulations, following exposure to the digestive process (* $p < 0.05$).

*Figure 5*. Cell viability of intestinal epithelia exposed to digestive fluids in the absence of the formulations (0%) and increasing concentrations of PEAMag® (A), normast® MPS microgranules (B) and PEA of the invention (C) for 3 h (*cell viability < 70%).

*Figure 6*. TEER of intestinal epithelia exposed to digestive fluids in the absence of the formulations (0%) and increasing concentrations of PEAMag® (A), normast® MPS microgranules (B) and PEA of the invention (C) for 3 h.

*Figure 7*. Cell viability (A), Papp (B) and TEER trend (C) of intestinal epithelia exposed to digestive fluids in the absence of the formulations (Ctrl) and the formulations PEAMag®, normast® MPS microgranules and PEA of the invention for 3 h.

*Figure 8*. Structure of Palmitoylethanolamide (PEA).

*Figure 9*. The micronized PEA appears in crystalline form with more important diffraction peaks at 5.409°, 7.089°, 10.596°, 21.702° and 23.017° of the 2Theta angle. The diffraction peaks observed correspond to those reported in the literature. The PEA of the invention is presented in predominantly amorphous form, however, the presence of diffraction peaks attributable to the crystalline PEA is noted.

*Figure 10*. Left: Dispersion of 500 mg as such in 100 ml tap water, subsequently dispersed in GSF and FaSSIF V2 (Simulated gastric liquid and Simulated enteric liquid under fasting conditions, at 37°C and 250 rpm, magnetic stirring). Right: Dispersion of 500 mg of PEA conveyed in the composition of the invention (powder) in 100 ml of tap water, subsequently dispersed in GSF and FaSSIF V2 (Simulated gastric liquid and Simulated enteric liquid under fasting conditions, at 37°C and 250 rpm, magnetic stirring). It is possible to appreciate the complete dispersion of the PEA conveyed in the composition of the invention on the right and the emergence of the PEA on the surface of the liquid on the left.

## DETAILED DESCRIPTION OF THE INVENTION

*Solid composition in powder form.*

As mentioned above, the object of the present invention is a solid composition, in powder form, comprising palmitoylethanolamide (PEA) as an active ingredient, the PEA being conveyed in a self-emulsifying lipid matrix, comprising a lipid-surfactant phase (a) and a hydrophilic phase in the form of granules (b), as defined by the claims.

For the purposes of the present invention "solid" composition i powder form means a powder suitable for filling hard capsules.

By powder for filling hard capsules, it is intended a powder with flowability features and chemical-physical parameters such as to be correctly processed in the machinery adapted to fill hard capsules.

The solid composition object of the present invention is preferably used for the preparation of products for conventional oral use, such as single-dose granules and animal or vegetable gelatin capsules, in which the solid composition is preferably associated with suitable excipients and/or diluents.

Suitable excipients and diluents for the preparation of oral products based on the solid composition are, by way of non-limiting illustration, flavourings, sweeteners, pH correctors, colouring agents, adjuvants for the manufacture of powders (e.g., amorphous silica, calcium phosphate, magnesium stearate).

It should be noted that the composition object of the present invention is not suitable for compression and therefore for the formation of compresses/tablets.

For the purposes of the present invention, self-emulsifying lipid matrix is intended as a mixture consisting mainly of lipophilic molecules, comprising components with surfactant properties, capable of spontaneously emulsifying in a physiological aqueous medium, at body temperature ($> 36°C$). It should be noted that, preferably, the self-emulsifying lipid matrix contained in the composition of the present invention, spontaneously emulsifies already in gastric fluids (pH $\geq$ 1.2-1.6).

According to a preferred embodiment, the solid composition is for use as a medicament, nutritional supplement or food for special medical purposes (FSMP).

Still preferably, the composition is intended for use in the treatment of inflammatory or algesic states. The composition of the invention can therefore be used as an anti-inflammatory or analgesic.

Still preferably, the composition is intended for use in the treatment of inflammatory conditions in the course of neuritis; chronic pain; ongoing or post-herpetic neuropathic pain; compressive lumbosciatalgia; painful muscle and/or visceral syndromes; inflammatory muscle-tendinous diseases; painful muscle contracture syndromes; pelvic inflammatory syndromes; vulvodynia; inflammatory and painful conditions affecting the female reproductive system; dental pain or post-operative dental surgery; pain in the course of osteoarthritis; post-surgical spinal pain; idiopathic or cancer pain.

Preferably, the solid composition can be used for both human and animal use.

The solid composition is preferably intended for oral intake as is or after dispersion in water.

It should be noted that, advantageously, the solid composition for the above-mentioned use, is characterized by a bioaccessibility of the PEA between 58% and 62% by weight on the weight of the PEA contained in a dosage unit.

Still preferably, the solid composition of the invention is in powder form, said powder being characterized by particle sizes between 565 nm and 1,233 nm, measured with Dynamic Light Scattering (DLS).

Preferably, the powder in which the solid composition of the invention is formed is characterized by a polydispersity index (PDI) between 0.356 and 0.514, measured with DLS.

According to a preferred embodiment, the solid composition of the invention comprises, in addition to the PEA, also other active ingredients, preferably with contracture-relieving and neuro-trophic action. Active ingredients with a contracture-relieving and neuro-trophic action suitable for conveyance in the composition of the invention are Vitamins of the B group such as Vitamin B1, B6 and B12; sulphurated organic acids such as $\alpha$-Lipoic acid; Magnesium oxide, Magnesium chloride, Magnesium gluconate, Magnesium citrate or other organic salts of Magnesium or combinations of the foregoing. The use of organic salts of Magnesium in combination with PEA is known in the prior art (Italian Patent No. 102019000005370, entitled *"Nutraceutical and pharmaceutical composition for the treatment of pain disorders"):* the interaction of PEA with the CB1 and CB2 receptors is of particular importance, since it enhances the effect of the endogenous molecules which the body naturally produces in response to an algal stimulus, in order to protect the CNS from over-excitation or hypersensitivity phenomena, only in the anatomical districts where the painful stimulus was generated. In particular, PEA acts by increasing the effect of endogenous ligands on the CB1 and CB2 receptors; such receptors centrally cause a retrograde blockade of neuronal transmission and the production of anti-inflammatory cytokines at the microglial level. It is thereby possible to exploit the powerful pain-relieving effect of endocannabinoids, avoiding their psychotropic effects. Magnesium salts act on cannabinoid receptors by increasing the activity of endogenous substances; in fact, magnesium increases the affinity of endogenous cannabinoids with an antipain and anti-inflammatory action on the CB1 and CB2 receptors. Furthermore, by blocking the transmission of the pain pulse, magnesium acts as an amplifier of the pain relief effect.

It should be noted that such further active ingredients can be conveyed in the lipid-surfactant phase (*a*) together with the PEA or, alternatively, in the hydrophilic phase in the form of granules (*b*).

According to a preferred embodiment, the solid composition of the invention is formulated in administration units comprising 3 or 4 grams of the powder composition.

The solid composition comprises two steps:

a) a lipid-surfactant phase including the PEA;

b) a hydrophilic phase in the form of granules containing or consisting of glucose polymers and/or polyols, on which the lipid-surfactant phase is adsorbed.

The two aforementioned phases (a and b) form the self-emulsifying lipid matrix. Preferably, the self-emulsifying lipid matrix is exclusively formed by the lipid-surfactant phase which includes the PEA (a) and the hydrophilic phase in the form of granules (b) which contains or consists of glucose polymers and/or polyols, on which the lipid-surfactant phase is adsorbed.

It should be noted that, preferably, the solid composition does not comprise ascorbyl palmitate.

The features of the lipid-surfactant phase (a) and the hydrophilic powder phase (b) are described separately below. The fact that the two phases are described separately only serves the purpose of simplifying the presentation of the information, but it is not indicative of a separate existence thereof in the solid composition of the invention.

_Lipid-surfactant phase (a)._

The lipid-surfactant phase comprises or consists of

- Palmitoylethanolamide (PEA)

- at least one medium chain triglyceride (MTC)

- at least one mono and diglyceride of fatty acids

- at least one mono- or poly-unsaturated fatty acid, or a precursor thereof

- at least one hydrophilic surfactant, as defined by the claims.

According to a preferred embodiment, the lipid-surfactant phase (a) comprises mono and diglycerides of fatty acids in a concentration between 10% and 20%, preferably between 10% and 18%, preferably between 12% and 16% by weight, on the total weight of the solid composition (w/w).

It should be noted that by mono and diglycerides of fatty acids preferably it is meant a mixture of mono- and diglycerides of fatty acids having a number of carbon atoms between 12 and 20 (C12-C20). Preferably, the mono and diglycerides of fatty acids are characterized by C12-C20 saturated fatty acids or C12-C20 unsaturated fatty acids or by mixtures of C12-C20 saturated and unsaturated fatty acids.

The mixture of mono and diglycerides of fatty acids known, at the dietary level, with the identifier E471 is suitable for the purposes of the invention. Other examples of mono and diglycerides of fatty acids useful in the objects of the invention are glyceric esters of stearic acid, glyceric esters of palmitic acid, glyceric esters of oleic acid or mixtures of the foregoing.

It should be noted that commercially available ingredients which can be classified as "mono and diglycerides of fatty acids" may contain fractions or traces of _tri_-glycerides of fatty acids. For example, the additive E471 contains glycerol mono-esters in a concentration ranging from 93% to 42%, di-esters in a concentration ranging from 4% to 44%, tri-esters in a concentration ranging from 0% to 8% by weight on the total weight of the additive (EFSA FAF Panel (EFSA Panel on Food Additives and Flavourings), Younes, M, Aquilina, G, Castle, L, Engel, K-H, Fowler, P, Frutos Fernandez, MJ, Fürst, P, Giirtler, R, Husøy, T, Manco, M, Mennes, W, Moldeus, P, Passamonti, S, Shah, R, Waalkens-Berendsen, I, Wölfle, D, Wright, M, Dusemund, B, Mortensen, A, Turck, D, Barmaz, S, Tard, A, Vianello, G and Gundert-Remy, U, 2021. Scientific opinion on the re-evaluation of mono- and diglycerides of fatty acids (E 471) as food additive in foods for infants below 16 weeks of age and follow-up of their re-evaluation as food additives for uses in foods for all population groups. EFSA Journal 2021;19(11):6885, 44 pp.)

Generally, the mono and diglycerides of fatty acids are ingredients in which the possible percentage of glycerol triesters is less than 50%, preferably less than 40%, preferably less than 25%, preferably less than 10% by weight on the total weight of the ingredient.

Preferably, the palmitoylethanolamide (PEA) is in a concentration between 15% and 25%, preferably between 15% and 20%, preferably between 15% and 18% (w/w).

Still preferably, the PEA delivered in the composition of the invention is in ground form; the PEA conveyed in the composition of the invention is neither in micronized form nor in ultra-micronized form.

Preferably, the solid composition comprises the at least one medium chain triglyceride in a concentration between 1% and 10%, preferably between 1% and 8%, preferably between 2% and 8% (w/w).

It should be noted that medium chain triglyceride means the tri-ester of glycerol with medium chain fatty acids, i.e., C6-12.

According to a preferred embodiment, the at least one medium chain triglyceride comprises esterifications obtained with saturated fatty acids, preferably belonging to the group consisting of: capronic acid (hexanoic), enanthic acid (heptanoic), caprylic acid (octanoic), pelargonic acid (nonanoic), capric acid (decanoic), undecanoic acid, lauric acid (dodecanoic).

Still preferably, the at least one medium chain triglyceride is a mixture of medium chain triglycerides, said medium chain triglycerides comprising esterifications obtained with the fatty acids belonging to the group consisting of: capronic acid (hexanoic acid), enanthic acid (heptanoic acid), caprylic acid (octanoic acid), pelargonic acid (nonanoic acid), capric acid (decanoic acid), undecanoic acid, lauric acid (dodecanoic acid) and mixtures of the foregoing.

Still preferably, the preferred at least one medium chain triglyceride is the mixture of capric and caprylic triglyceride.

Preferably, the at least one mono- or poly-unsaturated fatty acid, or a derivative thereof, is in a concentration between 1% and 10%, preferably between 1% and 8%, preferably between 1% and 5%, preferably between 1% and 3% (w/w).

The at least one mono- or poly-unsaturated fatty acid can be included in the composition of the invention either in the form of glycerol ester, or in the form of (free) carboxylic acid.

Still preferably, the glycerol ester comprising the mono- or poly-unsaturated fatty acid is a triglyceride, the triglyceride preferably comprising mono- or poly-unsaturated fatty acids selected from the fatty acids having a carbon number between 16 and 22 (C16-C22). Preferably, the mono- or poly-unsaturated fatty acids esterifying glycerol are selected from the group consisting of: oleic acid (Omega-9), linoleic acid (Omega-6), eicosapentaenoic acid (Omega-3), docosahexanoic acid (Omega-3).

It should be noted that the triglyceride form of the mono- or poly-unsaturated fatty acid forms a precursor of the fatty acid contained therein, which will be released *in vivo* by esterases.

Alternatively, the mono- or poly-unsaturated fatty acid in the form of carboxylic acid is preferably selected from the fatty acids having a carbon number between 16 and 22 (C16-C22). Preferably, when the fatty acid is poly-unsaturated, the number of unsaturations is between 2 and 6. Preferably, the mono- or poly-unsaturated fatty acids are selected from the group consisting of: oleic acid (Omega-9), linoleic acid (Omega-6), eicosapentaenoic acid (Omega-3), docosahexanoic acid (Omega-3).

The preferred embodiment of the invention envisages that the mono- or poly-unsaturated fatty acid is in carboxylic form. Still preferably, the mono- or poly-unsaturated fatty acid in carboxylic form is selected from oleic acid and conjugated linoleic acid.

Regardless of the form in which the mono- or poly-unsaturated fatty acid is included in the composition of the invention, the weight ratio between the mono- or poly-unsaturated fatty acid content and the PEA is between 1:6 and 1:10. The Applicant considers such a ration functional to ensure that the PEA, conveyed in the composition of the invention, is emulsified in vivo in contact with gastro-enteric liquids.

Preferably, the at least one hydrophilic surfactant is in a concentration between 2% and 10%, preferably between 2% and 8%, preferably between 2% and 5% (w/w).

According to a preferred embodiment, the hydrophilic surfactant is a high HLB (> 9) hydrophilic surfactant (HLB: Hydrophilic-Lipophilic Balance).

Said at least one hydrophilic surfactant is preferably selected from polyoxyethylene sorbitan esters with a C12-C20 fatty acid, sucrose esters with a C12-C20 fatty acid (sucrester) or mixtures of the two.

The preferred hydrophilic surfactants in the objects of the invention are polyoxyethylene sorbitan esters C12-C20, where the C12-C20 fatty acids can be chosen from saturated or unsaturated fatty acids.

Still preferably, the polyoxyethylene sorbitan esters with a C12-C20 fatty acid are polysorbate 20 and polysorbate 80.

According to a preferred embodiment, the lipid-surfactant phase, as defined by the claims, comprises or consists of

- Palmitoylethanolamide (PEA) in a concentration between 15% and 25% (w/w);
- at least one medium-chain triglyceride (MTC) in a concentration between 1% and 10%;
- at least one mono and diglyceride of fatty acids in a concentration between 10% and 20% (w/w);
- at least one mono- or poly-unsaturated fatty acid is in a concentration between 1% and 10% (w/w);
- at least one hydrophilic surfactant in a concentration between 2% and 10% (w/w).

*Hydrophilic phase in granule form (b).*

As mentioned above, the solid composition of the invention, in addition to the lipid-surfactant phase (a), comprises a hydrophilic phase in granules (b) on which the lipid-surfactant phase (a) is adsorbed.

The hydrophilic phase in granules (b) comprises or consists of glucose polymers and/or polyols.

Preferably, the solid composition of the invention comprises the hydrophilic phase (b) in a concentration between 20% and 70%, preferably between 30% and 70%, preferably between 35% and 60%, preferably between 40% and 60% (w/w).

Preferably, the glucose polymers and/or polyols are selected from the group consisting of: maltodextrins, sorbitol, mannitol, isomalt. Still preferably, the hydrophilic phase in granules (b) consists of maltodextrins.

It should be noted that, according to a preferred embodiment, the lipid-surfactant phase (a) and the hydrophilic phase in granule form (b) are combined in a weight ratio between 0.4:1 and 1:1, preferably between 0.5:1 and 0.7:1.

*Process for producing the solid composition of the invention.*

A further object of the present invention is a process for the preparation of the solid composition, comprising the steps of:

i) preparing a lipid-surfactant mixture, as defined by the claims, comprising

- at least one medium chain triglyceride (MTC)
- at least one mono and diglyceride of fatty acids
- at least one mono- or poly-unsaturated fatty acid, or a precursor thereof
- at least one hydrophilic surfactant

ii) dissolving the lipid-surfactant mixture at a temperature between 70°C and 100°C, until obtaining a hot, liquid and transparent lipid-surfactant mixture;

iii) adding the PEA to the hot lipid-surfactant mixture, under stirring, until completely dissolved, to obtain the hot lipid-surfactant phase (a);

iv) preparing the hydrophilic phase in the form of granules (b) and adding the hot lipid-surfactant phase (a), under stirring, maintaining the temperature between 70°C and 100°C, so as to obtain a hot precursor of the solid composition, in powder form;

v) maintaining the hot precursor of the solid composition, in powder form, under stirring and cooling to room temperature (25°C-30°C), to obtain the solid composition, in powder form;

vi) sieving the solid composition, in powder form, until obtaining a particle size between 565 nm and 1,233 nm.

Preferably, the step of dissolving the lipid-surfactant mixture (ii) is conducted at a temperature between 80°C and 90°C.

According to a preferred embodiment, after the sieving step (vi), the solid composition, in powder form, is combined with suitable excipients which improve the flowability and make the particle size uniform.

The preferred excipients for this purpose includes amorphous silica.

Following the sieving step (vi) the solid composition can be added with further excipients and/or functional diluents to improve the chemical-physical features and/or organoleptic features of the product.

Its should be noted that the specifications of the constituent components of the surfactant-lipid mixture, the lipid-surfactant phase (a), the hydrophilic phase in granule form (b) are the same as those described in the preceding chapters of the present patent application.

**EXAMPLES**

By way of non-limiting illustration, two embodiment examples are given below of the solid composition, in powder form, object of the present invention.

*I. Example 1*

| Phase | Raw material | Chemical name | g / 3 g bag |
|---|---|---|---|
| A | Non-micronized ground PEA | Palmitoylethanolamide | 0.500 |
| A | KIRNOL | Mono- and diglycerides of fatty acids; E 471 | 0.400 |
| A | VEREMUL T 80 | Polysorbate 80 | 0.100 |
| A | LABRAFAC FG | Caprylic/Capric Triglyceride | 0.150 |
| A | TONALIN FFA 80 | Conjugated Linoeic Acid | 0.050 |
| B | GLUCIDEX | Maltodextrins | as needed 3 g |
| C | AMORPHOUS SILICA | Amorphous silica | 0.100 |
| D | CITRIC ACID | Citric acid | 0.020 |
| D | ORANGE FLAVOURING | Flavouring | 0.025 |
| D | SUCRALOSE | Sucralose | 0.003 |
| TOTAL | | | **3.000** |

*II. Example 2*

| Phase | Raw material | Chemical name | g / 4 g bag |
|---|---|---|---|
| A | Non-micronized ground PEA | Palmitoylethanolamide | 0.600 |
| A | KIRNOL | Mono- and diglycerides of fatty acids (E 471) | 0.600 |
| A | VEREMUL T 80 | Polysorbate 80 | 0.120 |
| A | LABRAFAC FG | Caprylic/Capric Triglyceride | 0.180 |
| A | TONALIN FFA 80 | Conjugated Linoeic Acid | 0.070 |
| B | GLUCIDEX | Maltodextrins | as needed 4 g |
| B | MAGNESIUM OXIDE (heavy powder) | Magnesium Oxide | 0.167 |
| C | AMORPHOUS SILICA | Amorphous silica | 0.130 |
| D | CITRIC ACID | Citric acid | 0.030 |
| D | ORANGE FLAVOURING | Flavouring | 0.025 |
| D | SUCRALOSE | Sucralose | 0.003 |
| TOTAL | | | **4.000** |

*III. Process for Preparing Examples 1 and 2*

**Phase A:** weigh the PEA. In glass beakers, successively weigh fatty acid mono and diglycerides, medium chain fatty acid triglyceride, hydrophilic surfactant and the conjugated fatty acid. Place the mixture to be heated on a thermostated heating plate at 85°C. Check the complete liquefaction of the mixture (totally transparent yellow liquid). Disperse the PEA in the melted mixture at 85°C and maintain the temperature until completely dissolved (stirring if necessary). A completely clear phase must be obtained without any undissolved aggregates.

**Step B:** mix the maltodextrins together with the MgO until a homogeneous and uniform powder is obtained (sieve).

Proceed to wet **Phase B** with Phase A at 85°C, under vigorous mechanical stirring (ideal horizontal or vertical screw kneader) until a wet powder (paste) is obtained. Continue mixing until powder temperature t < 30°C.

Combine **Phase C** (silica) with **Phase A+B** at room temperature under manual or mechanical stirring until a dry, homogeneous and flowable powder (granule) is obtained. Continue to sieving.

Combine **Phase A+B+C** with **Phase D** and mix until a fine, uniform, non-moist powder is obtained. Sieve.

### IV. Comparative study of the bioaccessibility and bioavailability of three Palmitoylethanolamide (PEA) based formulations

To verify the actual increase of oral bioaccessibility of PEA in the composition of the invention, with respect to a non-micronized powder form of PEA and in the form of micronized powder, an in vitro digestive simulation test was performed with simulated gastro-enteric liquids (SGF and SIF) to calculate the bioaccessible fraction of the three different forms of PEA and subsequently the bioaccessible fraction of the three powders was applied to a transwell-loaded CACO-2 cell model to measure the fraction thereof capable of overcoming the enteric epithelium with trans-cellular or para-cellular mechanism (A-BS) of the molecule.

### IV.1 OBJECTIVE

The purpose of the present study is to compare the bioaccessibility and intestinal absorption of PEA released by three formulations: (i) PEAMag® 1200; (ii) self-emulsifying PEA granules in accordance with the invention; and (iii) the normast® MPS reference formulation (EPITECH SpA).

### IV.2 MATERIALS AND METHODS

*IV.2.1 Sample description*

The bioaccessibility and bioavailability evaluation was performed on three PEA-based formulations, the details of which are shown in Table 1.

*Table 1.* Details of the formulations analysed.

| Sample | Order reference | Format | Dosage |
|---|---|---|---|
| PEA of the invention (Example 1 Composition) | 21LA06837 | Powder | 3 g/day |
| normast® MPS MICRO GRANULES (Ultra-micronized palmitoylethanolamide 600 mg; Micronized palmitoylethanolamide 300 mg; Fructose; Sweetener: sorbitol; Emulsifiers: sucrose palmitic esters, vegetable polysorbate, cross-linked sodium carboxymethylcellulose. | 21LA06833 | Powder | 1-2 sachets/-day |
| PEAMag® 1200 (Magnesium lactate, palmitoylethanolamide; bulking agent: sorbitol; maltodextrins, flavourings; acidity regulator: citric acid; anti-caking agent: silicon dioxide; sweetener: sucralose) | 21LA06835 | Powder | 1 stick/day |

*IV. 2.2 Determination of PEA content in the three formulations*

The PEA content of the three formulations was determined by HPLC analysis (High Performance Liquid Chromatography), whose LOD and LOQ values are 4.5 mg/L and 15 mg/L, respectively. The measured quantities were compared with the declared quantities and the PEA recovery was calculated as the ratio of the measured quantities to the declared quantities.

*IV.2.3 Determination of the particle size distribution of the three formulations*

The particle size distribution after re-suspension in digestive fluids was assessed by Dynamic Light Scattering (DLS) for all three formulations. The particle size distribution curves were obtained as an average of 5 instrument replicates as a function of signal intensity only. The conversion of the latter into the particle number or volume was not considered to avoid the propagation of errors which the calculation could produce, as such a calculation requires the use of additional parameters (e.g., the refractive index of the particles) which are not available.

*IV.2.4 Digestive process*

An amount of the three formulations corresponding to 600 mg of PEA was subjected to in vitro digestion process.

The latter is designed to simulate the human digestive process (divided into the oral, gastric and intestinal compartments) and is based on the use of fluids which simulate the physiological secretions typical of the digestive process (saliva, gastric juices, pancreatic juices and bile). At the end of the digestive process, the concentration of PEA was measured in the complete digest and compared with the declared titre to estimate the overall recovery of the process. After a centrifugation step (2750 g for 5 minutes)(1), the PEA was quantified in the pellet, equivalent to the excreted fraction, and in the supernatant, corresponding to the bioaccessible fraction. The PEA concentration in the samples was measured by HPLC.

*IV.2.5 In vitro model of intestinal epithelium*

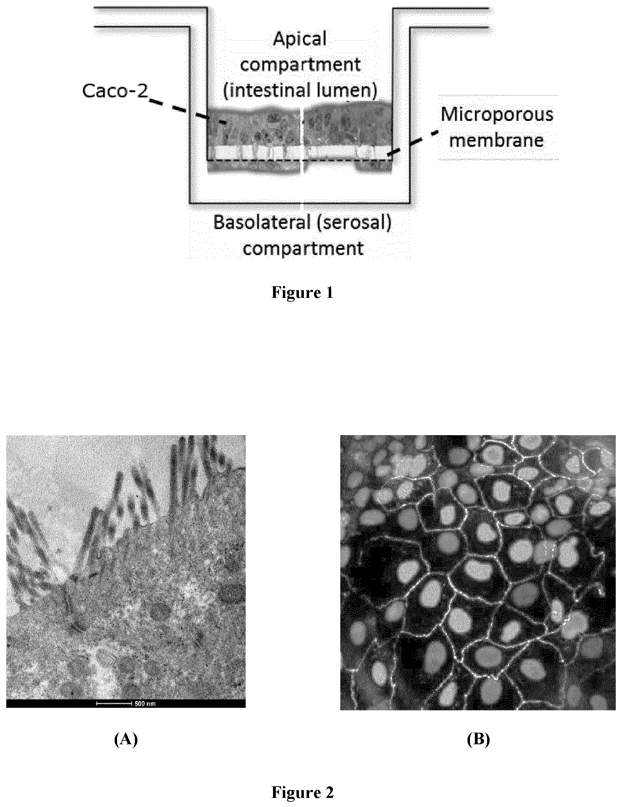

The intestinal absorption of the PEA present in the three formulations was determined using an in vitro human intestinal epithelium model, based on human Caco-2 intestinal adenocarcinoma cells (ATCC, HTB-37TM) organized as a functional monolayer on Transwell® inserts. Transwell® inserts are characterized by two compartments separated by a microporous membrane (Figure 1): apical (or luminal) and basolateral (or serosal). The Caco-2 monolayers grown on the microporous membranes consist of polarized cells with morpho-functional features typical of enterocytes, such as the presence of microvilli, tight junctions and p-glycoprotein (P-gp) (Figure 2).

*IV.2.6 Evaluation of the impact of the digested formulations on intestinal epithelium viability*

The impact of the digested formulations on intestinal epithelium viability was assessed by means of dose-response curve. As a result of the digestive process, the supernatant (bioaccessible fraction) resulting from the digestion of the formulations was diluted in digestive fluids. The different concentrations obtained (0 to 100% supernatant) were added to the apical compartment of the intestinal epithelium in vitro, while Hank's Balanced Salt Solution Buffer (HBSS) added with 1% BSA (Bovine Serum Albumin) was added in the basolateral compartment. As a negative control, the digestive fluids were used in the absence of the active ingredient. After 3 hours of incubation, the intestinal epithelia viability was assessed by MTS assay. The MTS assay is based on the reduction of the MTS tetrazolium compound by viable cells to generate the coloured product formazane, quantifiable by measuring the absorbance thereof at 490 nm. In parallel, the impact of the bioaccessible fractions of the analysed formulations on the barrier integrity of the intestinal epithelium was evaluated by measuring the transepithelial electrical resistance (TEER).

*IV.2.7 Evaluation of intestinal absorption of PEA*

At the end of the digestive process, the maximum non-toxic concentration of the bioaccessible fraction (supernatant) was added to the apical compartment of the intestinal epithelium in vitro. Digestive fluids lacking the active ingredient were used as a control, while HBSS added with 1% BSA was added to the basolateral compartment. After 3 hours of exposure, the apical and basolateral fractions were recovered and their PEA content was determined by HPLC analysis.

*IV.2.8 Vitality and integrity of the intestinal epithelial model barrier*

At the end of absorption, the cell viability and barrier integrity of the intestinal epithelium model were assessed. Cell viability was evaluated using the MTS assay, while barrier integrity was evaluated by measuring the transepithelial electrical resistance (TEER) of the cell monolayer and evaluating its permeability to Lucifer Yellow, a polar tracer unable to permeate through intact tight junctions. The apparent permeability coefficient (Papp, cm/min) was calculated with the following formula:

$$Papp = (\Delta C\, V)/(\Delta t\, A\, C0)$$

where $\Delta C/\Delta t$ is the flow of the molecule transported through the monolayer during the incubation time (mM/min), V is the volume of the basolateral compartment ($cm^3$), A is the membrane area ($cm^2$), C0 is the initial concentration of the Lucifer Yellow molecule in the apical compartment.

*IV.2.9 Statistical analysis*

All the data are presented as mean $\pm$ standard deviation (SD) of three independent experiments. A T-test analysis was performed to determine whether there were statistically significant differences between the different conditions. The T-test is a statistical method used to test the differences between the means of a series. The differences between the groups were considered significant at $p < 0.05$. All the statistical analyses were performed with OriginLab software.

**IV.3 RESULTS**

*IV.3.1 Determination of PEA titre in the three formulations*

The PEA content in the three formulations was measured by HPLC and compared with the PEA titre declared by the Client. As reported in Table 2, the measured titre is compatible with that declared for all three formulations, with a recovery of approximately 98.3%, 101.4% and 92.8% for PEAMag [®] 1200, normast[®] MPS microgranules and LiBADS PEA, respectively.

*Table 2.* Comparison of the measured titre and the declared titre of the three PEA formulations.

|  | Declared Titre (g PEA/ 100g formulation) | Measured Titre (g PEA/ 100g formulation) | Recovery (%) |
|---|---|---|---|
| **PEAMag [®] 1200** | 30 | 29.5 | 98.3 |
| **Normast [®] MPS microgranules** | 72 | 73 | 101.4 |
| **PEA of the invention** | 16.6 | 15.4 | 92.8 |

*IV.3.2 Determination of the particle size distribution of the three formulations*

The determination of the particle size distribution of the three formulations was performed by DLS analysis. In Figure 3 and Table 3 it can be seen that the normast[®] MPS microgranules have a significantly greater hydrodynamic radius with respect to the other two formulations, while the PEA of the invention and PEAMag[®] 1200 are characterized by particles with similar hydrodynamic dimensions. The low polydispersity indices (PDI) for the PEA of the invention and normast[®] MPS microgranules suggest that the data obtained are consistent while, for PEAMag[®], the measurements may differ by default from the actual hydrodynamic diameter. In fact, in the correlogram for the latter formulation, the presence of large particles/aggregates is noted.

*Table 3.* Intensity peaks and polydispersity index for the three formulations measured by DLS. The results are shown as mean ± standard deviation.

|  | Main peak (nm) | Secondary peak (nm) | PDI | R.D. % |
|---|---|---|---|---|
| **PEAMag [®] 1200** | 783.0 ± 108.1 |  | 0.512 ± 0.110 | 21.5 |
| **Normast [®] MPS microgranules** | 1696.0 ± 646.1 | 5207 ± 502.1 | 0.267 ± 0.083 | 31 |
| **PEA of the invention** | 899.1 ± 333.4 | 150.5 ± 50.0 | 0.435 ± 0.079 | 18.2 |

*IV.3.3 Release of PEA from the three formulations - bioaccessible fraction*

Bioaccessibility indicates the fraction of active ingredient released from the matrix during the digestive process and available for absorption. In order to quantify the bioaccessible fraction of PEA, i.e., the fraction released into the supernatant by the three formulations during the digestive process, an amount of the three formulations, corresponding to 600 mg of PEA, was subjected to the digestive process in vitro, at the end of which the recovery of PEA and the amount thereof in the bioaccessible fraction (supernatant) and excreted (pellet) was determined. At the end of digestion, a recovery of 98.8%, 101% and 77.9% of PEA is observed for PEAMag[®], normast[®] MPS microgranules and the PEA of the invention, respectively. As indicated by Figure 4A and Table 4, the release of PEA from PEAMag[®] and normast[®] MPS microgranules during the digestive process (bioaccessibility) is limited (1.1% and 0.9%, respectively), and most of the active ingredient is localized at the level of the excreted fraction (pellet) (98.7% and 98.3%, respectively) (Figure 4B and Table 5). By contrast, 60.3% of the PEA contained in the formulation of the invention is released into the supernatant at the end of the digestive process, indicating significantly higher bioaccessibility with respect to the other two formulations (Figure 4A and Table 4).

Table 4. Bioaccessible fraction of PEA of the three formulations, expressed as mg and percentage (%) of the total amount of PEA measured at the end of the digestive process. The results are expressed as mean ± standard deviation.

| | Bioaccessibility | |
|---|---|---|
| | PEA (mg) | PEA (%) |
| PEAMag ® 1200 | 6.6 ± 1.5 | 1.1 ± 0.1 |
| Normast ® MPS microgranules | 6.1 ± 0.0 | 0.9 ± 0.0 |
| PEA of the invention | 282.3 ± 8.5 | 60.3 ± **1.7** |

Table 5. Excreted fraction of PEA of the three formulations, expressed as mg and percentage (%) of the total amount of PEA measured at the end of the digestive process. The results are expressed as mean ± standard deviation.

| | Excreted fraction | |
|---|---|---|
| | PEA (mg) | PEA (%) |
| PEAMag ® 1200 | 583.4 ± 60.6 | 98.7 ± 4.2 |
| Normast ® MPS microgranules | 601.6 ± 6.5 | 98.3 ± 2.0 |
| PEA of the invention | 158.4 ± 8.1 | 33.9 ± 1.9 |

*IV.3.4 Impact of the digested formulations on intestinal epithelium viability*

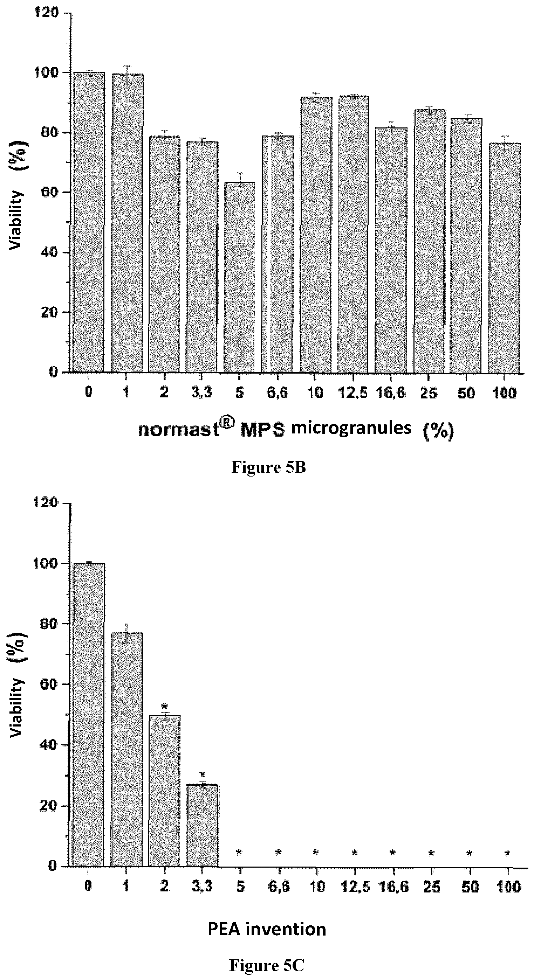

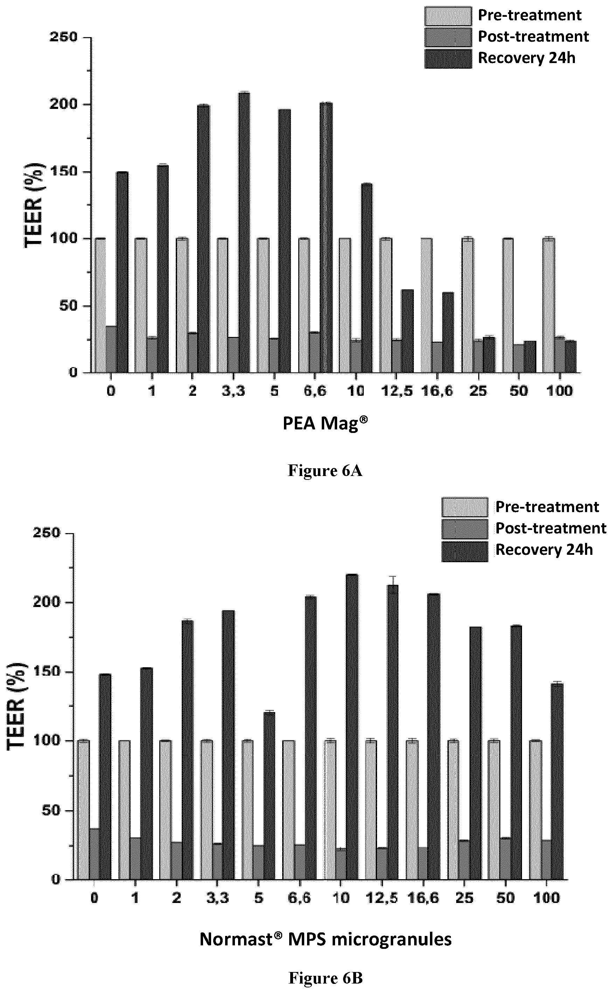

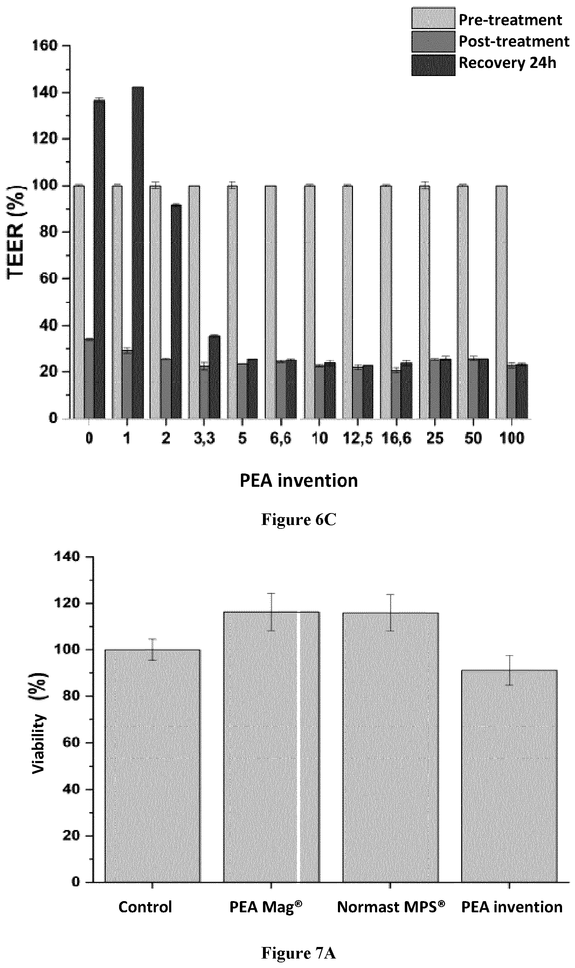

Prior to the evaluation of the bioavailability of PEA, the impact of the bioaccessible fraction of the three formulations on intestinal mucosa viability was evaluated. The Caco-2 monolayers were exposed to increasing concentrations of the bioaccessible fraction (from 0 to 100%) of the three formulations for 3 hours, obtaining the dose-response graphs shown in Figure 5A, B and C. From the dose response it emerges that, after 3 hours of incubation, the bioaccessible fractions as such of the three formulations significantly reduce intestinal epithelium viability. Consequently, for the evaluation of the absorption of PEA at the intestinal level, the Caco-2 monolayer was exposed to the maximum non-toxic concentration of the three formulations (1.3%), determined by combining the data obtained from the dose-response curves and the analysis of the transepithelial electrical resistance (TEER) (Figure 5 and 6).

*IV.3.5 Intestinal absorption of PEA*

In vitro intestinal epithelia were exposed to the maximum non-toxic concentration of the bioaccessible fraction of the three formulations for 3 hours. At the end of exposure, the amount of PEA was determined in both the apical (luminal) and basolateral (serosal) compartments. The absorption experiments show that after 3 hours of exposure, in the basolateral (bioavailable) fraction the PEA concentrations are lower than the LOQ (Limit of Quantification), equal to 15 mg/L, and LOD (Limit of Detection, 4.5 mg/L) for all three formulations. Considering the bioaccessibility values of the PEA conveyed in the technology of the invention and the dilution to which the bioaccessible fraction was subjected before absorption, a reduction of the amount of PEA present in the apical compartment at the end of the 3 h of incubation (20.3 μg measured with respect to an expected value of 99 μg) is observed. This reduction could correspond to the fraction of PEA absorbed, but not released in the basolateral compartment after 3 h. The low bioaccessibility values related to PEAMag® and normast® MPS microgranules do not allow to evaluate the reduction of PEA at the apical compartment as a possible indication of absorption.

*IV.3.6 Impact of digested formulations on intestinal mucosa viability and integrity*

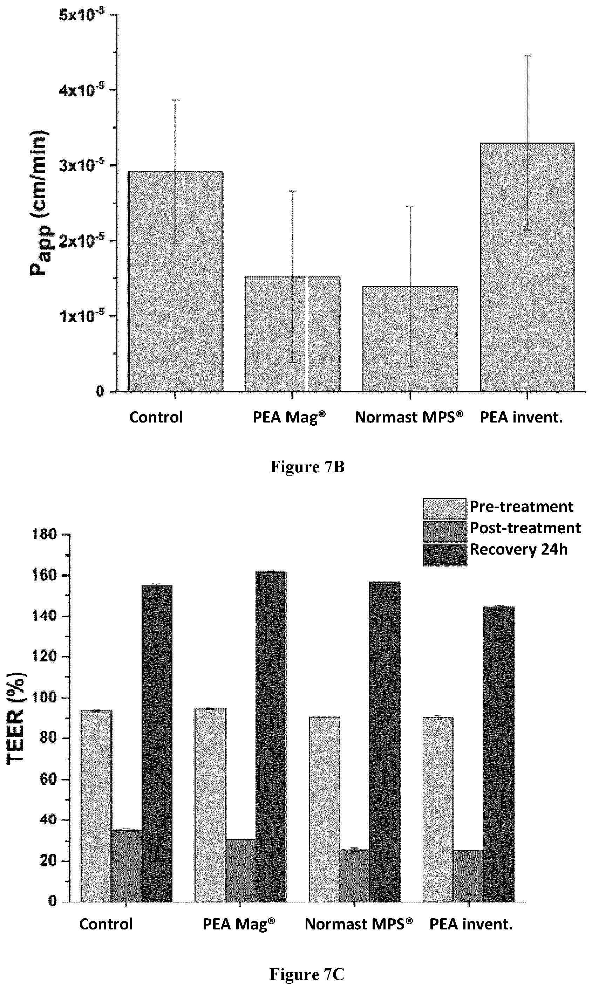

Parallel to absorption, the impact of the digested formulations on barrier vitality and integrity on the in vitro model of intestinal epithelium was evaluated. As can be seen from Figure 7, no significant alterations in the viability (Figure 7A) and apparent permeability (Figure 7B and 7C) of the intestinal epithelium were observed following treatment with the maximum non-toxic concentration of the bioaccessible fraction of the three formulations. Although the formulation, like intestinal fluids, increases permeability at the end of the treatment, such a variation is not permanent, as the transepithelial electrical resistance is recovered after 24 hours (Figure 7C).

## IV.4 CONCLUSIONS

In light of the results obtained, among the PEA-based formulations provided by the Client, the PEA of the invention is characterized by a higher bioaccessibility of PEA with respect to PEAMag® and normast® MPS microgranules. The reduction of PEA at the apical level in the epithelia treated with LiBADS PEA could suggest that the active ingredient is absorbed / internalized by the cells.

## IV.5 BIBLIOGRAPHY

1. Versantvoort CHM, Oomen AG, Van De Kamp E, Rompelberg CJM, Sips AJAM. Applicability of an in vitro digestion model in assessing the bioaccessibility of mycotoxins from food. Food Chem Toxicol. 2005;43(1):31-40.

## Claims

1. Solid composition, in powder form, comprising palmitoylethanolamide (PEA) as active ingredient,

   the solid composition being **characterized in that** it comprises

      a) a lipid-surfactant phase including the PEA and containing

         - at least one medium chain triglyceride (MTC)
         - at least one mono and diglyceride of fatty acids
         - at least one mono- or poly-unsaturated fatty acid, or precursors thereof
         - at least one hydrophilic surfactant

      b) a hydrophilic phase in the form of granules containing glucose polymers and/or polyols, on which the lipid-surfactant phase is adsorbed,

   wherein the weight ratio between the mono- or poly-unsaturated fatty acid content and the PEA is between 1:6 and 1:10.

2. Solid composition according to claim 1, wherein the at least one mono and diglyceride of fatty acids is in a concentration between 1% and 10% by weight on the total weight of the composition (w/w).

3. Solid composition according to claim 1 or 2, wherein the at least one mono- or polyunsaturated fatty acid is in the form of carboxylic acid.

4. Solid composition according to any one of claims 1 to 3, wherein the palmitoylethanolamide is in a concentration between 15% and 25% (w/w).

5. Solid composition according to any one of claims 1 to 4, wherein the at least one medium chain triglyceride is in a concentration between 1% and 10% (w/w).

6. Solid composition according to any one of claims 1 to 5, wherein the at least one hydrophilic surfactant is in a concentration between 2% and 10% (w/w).

7. Solid composition according to any one of claims 1 to 6, wherein the hydrophilic phase (*b*) is in a concentration between 20% and 70% (w/w).

8. Solid composition according to any one of claims 1 to 7, wherein the glucose polymers and/or polyols are selected from the group consisting of: maltodextrins, sorbitol, mannitol, isomalt.

9. Solid composition according to any one of claims 1 to 8, for human or animal use as a medicine or nutritional supplement or food for special medical purposes (FSMPs).

10. Solid composition for use according to claim 9, to be taken orally as such or after dispersion in water.

11. Solid composition for use according to claim 9 or 10, in the form of granules or hard animal or vegetable gelatin capsules.

12. Solid composition for use according to any one of claims 9 to 11, wherein the PEA is **characterized by** a bioaccessibility between 50% and 70% by weight on the PEA weight in a dosage unit.

13. Solid composition for use according to any one of claims 9 to 12 in the treatment of inflammatory or algesic states.

14. Solid composition for use according to any one of claims 9 to 13 in the treatment of inflammatory conditions in the course of neuritis; ongoing or post-herpetic neuropathic pain; painful muscle and/or visceral syndromes; inflammatory muscle-tendinous diseases; painful muscle contracture syndromes; pelvic inflammatory syndromes; vulvodynia; inflammatory and painful conditions affecting the female reproductive system; dental pain or post-operative dental surgery; pain in the course of osteoarthritis; post-surgical spinal pain; idiopathic or oncological pain.

15. Process for preparing the solid composition according to any one of claims 1 to 8 comprising the steps of:

    i) preparing a lipid-surfactant mixture comprising

        - at least one medium chain triglyceride (MTC)
        - at least one mono and diglyceride of fatty acids
        - at least one mono- or poly-unsaturated fatty acid
        - at least one hydrophilic surfactant

    ii) dissolving the lipid-surfactant mixture at a temperature between 70°C and 100°C, until a hot, liquid and transparent lipid-surfactant mixture is obtained;
    iii) adding the PEA to the hot lipid-surfactant mixture, under stirring, until completely dissolved, to obtain the hot lipid-surfactant phase (*a*);
    iv) preparing the hydrophilic phase in the form of granules (*b*) and adding the hot lipid- surfactant phase (*a*), under stirring, maintaining the temperature between 70°C and 100°C, to obtain a hot precursor of the solid composition, in powder form;
    v) maintaining the hot precursor of the solid composition, in powder form, under stirring and cooling to room temperature, to obtain the self-emulsifying lipid matrix, in powder form;

## Patentansprüche

1. Feste Zusammensetzung in Pulverform, umfassend Palmitoylethanolamid (PEA) als Wirkstoff, wobei die feste Zusammensetzung **dadurch gekennzeichnet ist, dass** sie umfasst:

    a) eine Lipid-Tensid-Phase, die das PEA einschließt und enthält:

        ◦ mindestens ein mittelkettiges Triglycerid (MTC)
        ◦ mindestens ein Mono- und Diglycerid von Fettsäuren
        ◦ mindestens eine mono- oder polyungesättigte Fettsäure oder Vorläufer davon
        ◦ mindestens ein hydrophiles Tensid

    b) eine hydrophile Phase in Form von Granulat, das Glucosepolymere und/oder Polyole enthält, auf der die Lipid-Tensid-Phase adsorbiert ist, **wobei** das Gewichtsverhältnis zwischen dem Gehalt an mono- oder polyungesättigter Fettsäure und dem PEA zwischen 1:6 und 1:10 liegt.

2. Feste Zusammensetzung nach Anspruch 1, wobei das mindestens ein Mono- und Diglycerid von Fettsäuren in einer Konzentration zwischen 1 Gew.-% und 10 Gew.-% bezogen auf das Gesamtgewicht der Zusammensetzung (w/w) vorliegt.

3. Feste Zusammensetzung nach Anspruch 1 oder 2, wobei die mindestens eine mono- oder polyungesättigte Fettsäure in Form von Carbonsäure vorliegt.

4. Feste Zusammensetzung nach einem der Ansprüche 1 bis 3, wobei das Palmitoylethanolamid in einer Konzentration zwischen 15 % und 25 % (w/w) vorliegt.

5. Feste Zusammensetzung nach einem der Ansprüche 1 bis 4, wobei das mindestens ein mittelkettiges Triglycerid in einer Konzentration zwischen 1 % und 10 % (w/w) vorliegt.

6. Feste Zusammensetzung nach einem der Ansprüche 1 bis 5, wobei das mindestens ein hydrophiles Tensid in einer Konzentration zwischen 2 % und 10 % (w/w) vorliegt.

7. Feste Zusammensetzung nach einem der Ansprüche 1 bis 6, wobei die hydrophile Phase (b) in einer Konzentration zwischen 20 % und 70 % (w/w) vorliegt.

8. Feste Zusammensetzung nach einem der Ansprüche 1 bis 7, wobei die Glucosepolymere und/oder Polyole ausgewählt sind aus der Gruppe bestehend aus: Maltodextrinen, Sorbit, Mannit, Isomalt.

9. Feste Zusammensetzung nach einem der Ansprüche 1 bis 8 zur Verwendung beim Menschen oder Tier als Arzneimittel oder Nahrungsergänzungsmittel oder Lebensmittel für besondere medizinische Zwecke (FSMPs).

10. Feste Zusammensetzung zur Verwendung nach Anspruch 9, zur oralen Einnahme als solche oder nach Dispersion in Wasser.

11. Feste Zusammensetzung zur Verwendung nach Anspruch 9 oder 10, in Form von Granulat oder tierischen oder pflanzlichen Hartgelatinekapseln.

12. Feste Zusammensetzung zur Verwendung nach einem der Ansprüche 9 bis 11, wobei das PEA durch eine Biozugänglichkeit zwischen 50 Gew.-% und 70 Gew.-% bezogen auf das PEA-Gewicht in einer Dosierungseinheit gekennzeichnet ist.

13. Feste Zusammensetzung zur Verwendung nach einem der Ansprüche 9 bis 12 bei der Behandlung von entzündlichen oder algetischen Zuständen.

14. Feste Zusammensetzung zur Verwendung nach einem der Ansprüche 9 bis 13 bei der Behandlung von entzündlichen Zuständen im Verlauf von Neuritis; anhaltendem oder postherpetischem neuropathischem Schmerz; schmerzhaften Muskel- und/oder Viszeralsyndromen; entzündlichen Muskel-Sehnen-Erkrankungen; schmerzhaften Muskelkontraktursyndromen; entzündlichen Beckensyndromen; Vulvodynie; entzündlichen und schmerzhaften Zuständen, die das weibliche Fortpflanzungssystem betreffen; Zahnschmerzen oder Schmerzen nach zahnärztlichen Eingriffen; Schmerzen im Verlauf von Osteoarthritis; postoperativen Wirbelsäulenschmerzen; idiopathischen oder onkologischen Schmerzen.

15. Verfahren zur Herstellung der festen Zusammensetzung nach einem der Ansprüche 1 bis 8, umfassend die Schritte:

   i) Herstellen einer Lipid-Tensid-Mischung, umfassend:

   ◦ mindestens ein mittelkettiges Triglycerid (MTC)
   ◦ mindestens ein Mono- und Diglycerid von Fettsäuren
   ◦ mindestens eine mono- oder polyungesättigte Fettsäure
   ◦ mindestens ein hydrophiles Tensid

   ii) Auflösen der Lipid-Tensid-Mischung bei einer Temperatur zwischen 70 °C und 100 °C, bis eine heiße, flüssige und transparente Lipid-Tensid-Mischung erhalten wird;
   iii) Zugeben des PEA zu der heißen Lipid-Tensid-Mischung unter Rühren, bis es vollständig aufgelöst ist, um die heiße Lipid-Tensid-Phase (a) zu erhalten;
   iv) Herstellen der hydrophilen Phase in Form von Granulat (b) und Zugeben der heißen Lipid-Tensid-Phase (a) unter Rühren, wobei die Temperatur zwischen 70 °C und 100 °C gehalten wird, um einen heißen Vorläufer der festen Zusammensetzung in Pulverform zu erhalten;
   v) Halten des heißen Vorläufers der festen Zusammensetzung in Pulverform unter Rühren und Abkühlen auf Raumtemperatur, um die selbstemulgierende Lipidmatrix in Pulverform zu erhalten.

**Revendications**

1. Composition solide, sous forme de poudre, comprenant de la palmitoyléthanolamide (PEA) en tant qu'ingrédient actif,

   la composition solide étant **caractérisée en ce qu'**elle comprend

      a) une phase lipide-tensioactif incluant la PEA et contenant

         ∘ au moins un triglycéride à chaîne moyenne (TCM)
         ∘ au moins un mono- et diglycéride d'acides gras
         ∘ au moins un acide gras mono- ou polyinsaturé, ou des précurseurs de celui-ci
         ∘ au moins un tensioactif hydrophile

      b) une phase hydrophile sous forme de granulés contenant des polymères de glucose et/ou des polyols, sur laquelle la phase lipide-tensioactif est adsorbée,

   **dans laquelle** le rapport pondéral entre la teneur en acide gras mono- ou polyinsaturé et la PEA est compris entre 1:6 et 1:10.

2. Composition solide selon la revendication 1, dans laquelle le au moins un mono- et diglycéride d'acides gras est à une concentration comprise entre 1 % et 10 % en poids par rapport au poids total de la composition (p/p).

3. Composition solide selon la revendication 1 ou 2, dans laquelle le au moins un acide gras mono- ou polyinsaturé est sous la forme d'acide carboxylique.

4. Composition solide selon l'une quelconque des revendications 1 à 3, dans laquelle la palmitoyléthanolamide est à une concentration comprise entre 15 % et 25 % (p/p).

5. Composition solide selon l'une quelconque des revendications 1 à 4, dans laquelle le au moins un triglycéride à chaîne moyenne est à une concentration comprise entre 1 % et 10 % (p/p).

6. Composition solide selon l'une quelconque des revendications 1 à 5, dans laquelle le au moins un tensioactif hydrophile est à une concentration comprise entre 2 % et 10 % (p/p).

7. Composition solide selon l'une quelconque des revendications 1 à 6, dans laquelle la phase hydrophile (b) est à une concentration comprise entre 20 % et 70 % (p/p).

8. Composition solide selon l'une quelconque des revendications 1 à 7, dans laquelle les polymères de glucose et/ou les polyols sont choisis dans le groupe consistant en : les maltodextrines, le sorbitol, le mannitol, l'isomalt.

9. Composition solide selon l'une quelconque des revendications 1 à 8, pour une utilisation humaine ou animale en tant que médicament ou complément nutritionnel ou aliment destiné à des fins médicales spéciales (ADDFMS).

10. Composition solide pour une utilisation selon la revendication 9, à prendre par voie orale telle quelle ou après dispersion dans l'eau.

11. Composition solide pour une utilisation selon la revendication 9 ou 10, sous la forme de granulés ou de gélules dures de gélatine animale ou végétale.

12. Composition solide pour une utilisation selon l'une quelconque des revendications 9 à 11, dans laquelle la PEA est **caractérisée par** une bioaccessibilité comprise entre 50 % et 70 % en poids par rapport au poids de PEA dans une unité de dosage.

13. Composition solide pour une utilisation selon l'une quelconque des revendications 9 à 12, dans le traitement d'états inflammatoires ou algiques.

14. Composition solide pour une utilisation selon l'une quelconque des revendications 9 à 13, dans le traitement de conditions inflammatoires au cours d'une névrite ; d'une douleur neuropathique persistante ou post-herpétique ; de syndromes douloureux musculaires et/ou viscéraux ; de maladies inflammatoires musculo-tendineuses ; de syndromes de contracture musculaire douloureuse ; de syndromes inflammatoires pelviens ; de la vulvodynie ; de conditions inflammatoires et douloureuses affectant le système reproducteur féminin ; d'une douleur dentaire ou d'une chirurgie dentaire post-opératoire ; d'une douleur au cours de l'arthrose ; d'une douleur spinale post-chirurgicale ; d'une douleur idiopathique ou oncologique.

15. Procédé de préparation de la composition solide selon l'une quelconque des revendications 1 à 8, comprenant les étapes consistant à :

> i) préparer un mélange lipide-tensioactif comprenant
>
> > ◦ au moins un triglycéride à chaîne moyenne (TCM)
> > ◦ au moins un mono- et diglycéride d'acides gras
> > ◦ au moins un acide gras mono- ou polyinsaturé
> > ◦ au moins un tensioactif hydrophile
>
> ii) dissoudre le mélange lipide-tensioactif à une température comprise entre 70 °C et 100 °C, jusqu'à l'obtention d'un mélange lipide-tensioactif chaud, liquide et transparent ;
> iii) ajouter la PEA au mélange lipide-tensioactif chaud, sous agitation, jusqu'à dissolution complète, pour obtenir la phase lipide-tensioactif chaude (a) ;
> iv) préparer la phase hydrophile sous forme de granulés (b) et y ajouter la phase lipide-tensioactif chaude (a), sous agitation, en maintenant la température entre 70 °C et 100 °C, pour obtenir un précurseur chaud de la composition solide, sous forme de poudre ;
> v) maintenir le précurseur chaud de la composition solide, sous forme de poudre, sous agitation et le refroidir à température ambiante, pour obtenir la matrice lipidique auto-émulsifiante, sous forme de poudre.

Figure 1

(A)

(B)

Figure 2

Figure 3

Figure 4A

Figure 4B

Figure 5A

Figure 5B

PEA invention

Figure 5C

Figure 6A

Figure 6B

Figure 6C

Figure 7A

**Figure 7B**

**Figure 7C**

Figure 8

Figure 9

Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017212447 A2

- IT 102019000005370

**Non-patent literature cited in the description**

- **GODLEWSKI G.** *Prostaglandins & Other Lipid Mediators.*, vol. 89 (3-4), 105-11
- **LINDA GABRIELSSON et al.** *Br J Clin Pharmacol*, 2016, vol. 82, 932-942
- **IMPELLIZZERI et al.** *J Neuroinflammation.*, 28 August 2014, vol. 11, 136
- **EMANUELA STOCHINO LOI et al.** *Int J Womens Health.*, 12 August 2019, vol. 11, 443-449

- **YOUNES, M** ; **AQUILINA, G** ; **CASTLE, L** ; **ENGEL, K-H** ; **FOWLER, P** ; **FRUTOS FERNANDEZ, MJ** ; **FÜRST, P** ; **GIIRTLER, R** ; **HUSØY, T** ; **MANCO, M**. Scientific opinion on the re-evaluation of mono- and diglycerides of fatty acids (E 471) as food additive in foods for infants below 16 weeks of age and follow-up of their re-evaluation as food additives for uses in foods for all population groups.. *EFSA Journal*, 2021, vol. 19 (11), 44
- **VERSANTVOORT CHM** ; **OOMEN AG** ; **VAN DE KAMP E** ; **ROMPELBERG CJM**. Sips AJAM. Applicability of an in vitro digestion model in assessing the bioaccessibility of mycotoxins from food.. *Food Chem Toxicol.*, 2005, vol. 43 (1), 31-40